(19) Europäisches Patentamt
European Patent Office
Office européen des brevets

(11) Numéro de publication: **0 397 567 A1**

(12) # DEMANDE DE BREVET EUROPEEN

(21) Numéro de dépôt: **90401236.6**

(22) Date de dépôt: **10.05.90**

(51) Int. Cl.5: **C07D 233/88, A61K 35/56, A61K 31/415**

(30) Priorité: **12.05.89 FR 8906249**

(43) Date de publication de la demande: **14.11.90 Bulletin 90/46**

(84) Etats contractants désignés: **AT BE CH DE DK ES FR GB GR IT LI LU NL SE**

(71) Demandeur: **RHONE-POULENC SANTE**
**20, avenue Raymond Aron**
**F-92160 Antony(FR)**

(72) Inventeur: **Commercon, Alain**
**1 bis rue Charles Floquet**
**F-94400 Vitry Sur Seine(FR)**
Inventeur: **Cousin, Jacky**
**61 rue Letellier**
**F-75015 Paris(FR)**
Inventeur: **Gueremy, Claude**
**3 rue Daumesnil**
**F-78800 Houilles(FR)**
Inventeur: **Ponsinet, Gérard**
**7 rue de Grand-Champ**
**F-94370 Sucy en Brie(FR)**

(74) Mandataire: **Pilard, Jacques et al**
**RHONE-POULENC SANTE, Service Brevets,**
**20 Avenue Raymond Aron**
**F-92165 Antony Cédex(FR)**

(54) **Nouvelle substance antitumorale, un procédé de préparation et les compositions pharmaceutiques qui la contiennent.**

(57) Le racémique thréo du produit de formule :

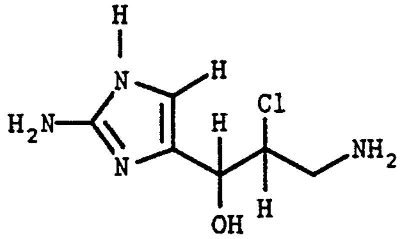

ses sels, son procédé de préparation et les compositions pharmaceutiques qui le contiennent.

EP 0 397 567 A1

## NOUVELLE SUBSTANCE ANTITUMORALE, UN PROCEDE DE PREPARATION ET LES COMPOSITIONS PHARMACEUTIQUES QUI LA CONTIENNENT

La présente invention concerne une nouvelle substance antitumorale, son procédé de préparation et les compositions pharmaceutiques qui la contiennent.

Dans le brevet français FR 85 10997 (2 585 020) a été décrite une substance biologiquement active, désignée sous le nom de girolline, extraite d'une éponge identifiée à Pseudaxinyssa cantharella, qui présente des propriétés antitumorales remarquables.

La structure de la girolline, sous forme de base ou de sel, peut être représentée par la formule suivante :

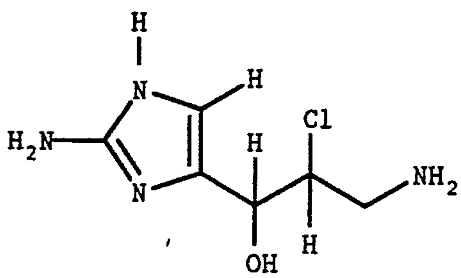
,
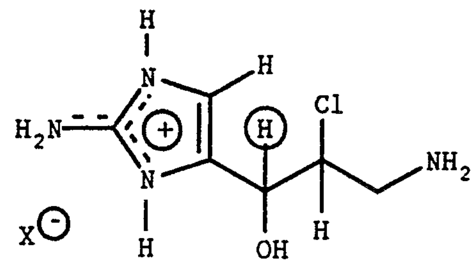

(I)

dans laquelle $X^-$ représente un anion tel que l'anion chlorure ($Cl^-$).

Un produit correspondant à la structure donnée ci-dessus, compte tenu de la présence de deux atomes de carbone asymétriques, peut exister sous forme de 4 isomères qui correspondent aux énantiomères des racémiques érythro et thréo.

Les études effectuées sur la girolline montrent que le produit d'origine naturelle correspond à l'un des énantiomères du racémique thréo.

Il a également été montré que le racémique thréo conserve les propriétés antitumorales remarquables de la girolline et que le racémique érythro et l'autre énantiomère du racémique thréo sont totalement inactifs.

La présente invention a pour objet le racémique thréo (1RS,2RS) de l'amino-3 (amino-2 1H-imidazolyl-4)-1 chloro-2 propanol-1 de formule (I), son procédé de préparation, les compositions pharmaceutiques qui le contiennent et son emploi comme intermédiaire pour la préparation d'un produit identique à la girolline naturelle.

Selon la présente invention, le racémique thréo du produit de formule (I) peut être obtenu par réduction d'un dérivé azo racémique thréo de formule générale :

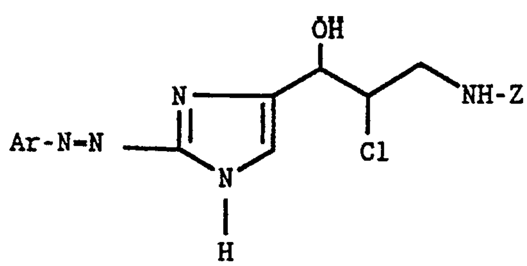

(II)

éventuellement sous forme de sel, conduisant au produit racémique thréo de formule générale :

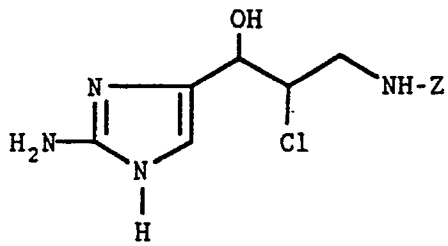

(III)

éventuellement sous forme de sel, dont le groupement protecteur Z est ensuite remplacé par un atome d'hydrogène.

Dans la formule générale (II), Ar représente un radical phényle éventuellement substitué par un ou plusieurs radicaux, identiques ou différents, choisis parmi les atomes d'halogène (fluor, chlore, brome) et les radicaux alcoyles contenant 1 à 4 atomes de carbone ou alkoxycarbonyles dont la partie alcoyle contient 1 à 4 atomes de carbone ou nitro.

Dans les formules générales (II) et (III), Z représente un groupement protecteur de la fonction amine facilement remplaçable par un atome d'hydrogène par hydrolyse en milieu acide. D'un intérêt tout particulier sont les produits de formule générale (II) ou (III) dans laquelle Z représente le radical formyle, un radical acyle ou alkoxycarbonyle dont la partie alkyle contient 1 à 4 atomes de carbone, éventuellement substitué par un radical phényle. De préférence Z représente le radical tert.butoxycarbonyle ou le radical formyle.

Généralement la réduction du produit de formule générale (II) est effectuée au moyen d'hydrogène en présence d'un catalyseur tel que l'oxyde de platine en opérant dans un solvant organique tel qu'un alcool (méthanol, éthanol) à une température comprise de préférence entre 0 et 30 °C. Il n'est pas nécessaire d'opérer sous pression.

Le remplacement du groupement protecteur Z par un atome d'hydrogène s'effectue généralement en milieu acide en opérant dans l'eau ou un alcool à une température comprise de préférence entre 0 et 50 °C. Comme acides peuvent être utilisés des acides minéraux, tels que les acides chlorhydrique ou sulfurique.

Comme alcools peuvent être utilisés le méthanol, l'éthanol, le propanol ou l'isopropanol.

Le racémique thréo du produit de formule générale (II) peut être obtenu par action d'un sel de diazonium de formule générale:

$Ar-N_2^+ , X^-$ (IV)

dans laquelle Ar est défini comme précédemment et $X^-$ représente un anion tel qu'un ion chlorure ou tétrafluoroborure, éventuellement préparé in situ, sur le racémique thréo du produit de formule générale:

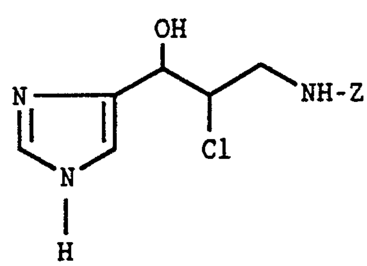

(V)

dans laquelle Z est défini comme précédemment.

La diazotation est généralement effectuée à une température voisine de 0 °C en opérant dans une solution aqueuse basique diluée telle qu'une solution aqueuse de carbonate de sodium.

Le produit racémique thréo de formule générale (V) peut être obtenu à partir d'un produit racémique thréo de formule générale:

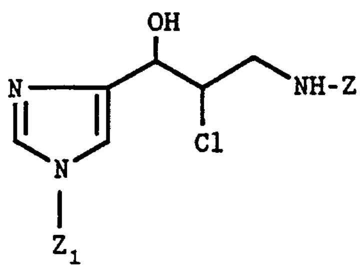

(VI)

dans laquelle Z est défini comme précédemment et $Z_1$ représente un groupement protecteur qui peut être éliminé sélectivement sans toucher au groupement protecteur Z. D'un intérêt tout particulier est le produit de formule générale (VI) dans laquelle $Z_1$ représente un radical trityle qui peut être éliminé en milieu alcoolique à une température généralement comprise entre 30 et 100 °C. Les alcools qui conviennent particulièrement bien sont choisis parmi le méthanol, l'éthanol, le propanol, l'isopropanol, le butanol et le tert.butanol. Dans le but d'accélérer la réaction, il peut être avantageux d'opérer en présence d'un acide organique tel que l'acide acétique, l'acide formique, l'acide benzoïque ou l'acide paratoluènesulfonique.

Le racémique thréo du produit de formule générale (VI) peut être obtenu par réduction d'un produit de formule générale :

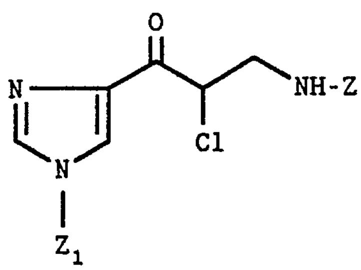

(VII)

dans laquelle Z et $Z_1$ sont définis comme précédemment.

Généralement la réduction est effectuée au moyen d'un borohydrure permettant d'obtenir un bon excès diastéréoisomérique. De préférence, on utilise le tri-sec.butylborohydrure de lithium dans un solvant organique choisi parmi les éthers tel que le tétrahydrofuranne à une température généralement inférieure à 0 °C et de préférence voisine de -75 °C. Cependant les borohydrures des métaux alcalins (sodium, potassium, lithium) en milieu alcoolique (éthanol) conduisent à des résultats satisfaisants.

Le produit de formule générale (VII) peut être obtenu par oxydation du racémique érythro d'un produit de formule générale :

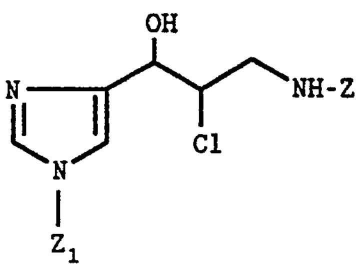

(VIII)

dans laquelle Z et $Z_1$ sont définis comme précédemment.

Généralement l'oxydation est effectuée par le dioxyde de manganèse en opérant dans un solvant organique inerte tel que le chlorure de méthylène à une température comprise entre 50 et 80 °C.

Le racémique érythro du produit de formule générale (VIII) peut être obtenu selon les méthodes habituelles de blocage de la fonction amine du racémique érythro du produit de formule générale :

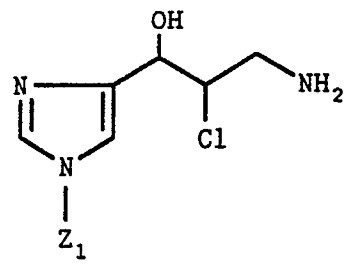

(IX)

dans laquelle $Z_1$ est défini comme précédemment.

Il est particulièrement avantageux d'utiliser le di-tert.butyldicarbonate pour introduire comme groupement protecteur le groupement tert.butoxycarbonyle.

Généralement, la réaction est effectuée dans un solvant organique inerte tel que le chlorure de méthylène à une température voisine de 20 °C.

Le racémique érythro du produit de formule générale (IX) peut être obtenu par ouverture en milieu acide suivie d'alcalinisation d'un produit racémique trans de formule générale :

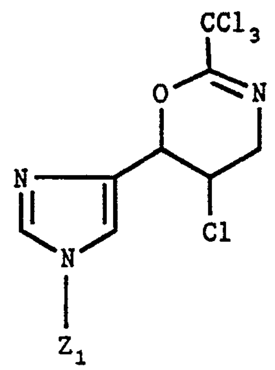

(X)

dans laquelle $Z_1$ est défini comme précédemment.

L'ouverture en milieu acide est généralement effectuée en opérant dans un solvant organique inerte tel qu'un éther (éther éthylique, éther diisopropylique) en présence d'un acide minéral fort tel que l'acide chlorhydrique ou l'acide sulfurique à une température voisine de 20 °C.

L'alcalinisation est généralement effectuée in situ par addition d'une base minérale telle que la soude, le carbonate de sodium, le bicarbonate de sodium, la potasse ou l'ammoniaque en maintenant la température inférieure à 10 °C.

Le racémique trans du produit de formule générale (X) peut être obtenu par action de l'hypochlorite de calcium en présence d'anhydride carbonique en milieu hydroorganique sur le produit de formule générale :

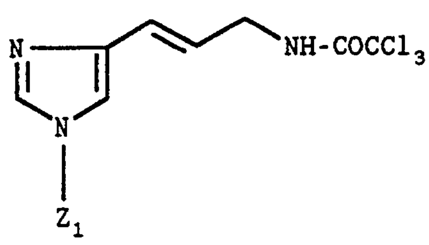

(XI)

dans laquelle $Z_1$ est défini comme précédemment.

La réaction peut aussi être réalisée en remplaçant l'hypochlorite de calcium par le complexe chlore-pyridine ou un agent de chloration tel que le N-chlorosuccinimide.

Le produit de formule générale (XI) peut être obtenu par action d'une hydrazine telle que la méthylhydrazine ou de l'hydroxylamine sur un phtalimide de formule générale :

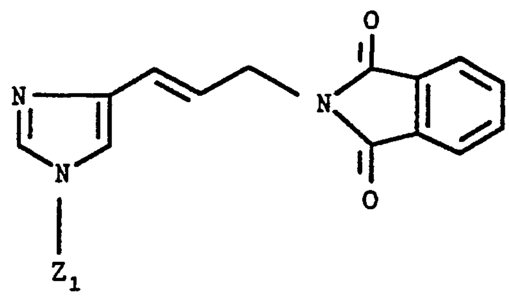

(XII)

dans laquelle $Z_1$ est défini comme précédemment, puis trichloracétylation du produit amino-allylique intermédiairement formé de formule générale :

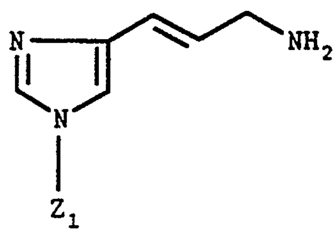

(XIII)

dans laquelle $Z_1$ est défini comme précédemment, par un agent d'acylation approprié tel que l'hexachloroacétone ou le chlorure de l'acide trichloracétique en présence d'une base.

Le produit de formule générale (XII) peut être obtenu selon le procédé décrit par G. de Nanteuil et al., Bull. Soc. Chim. France, 813 (1986).

Le produit de formule générale (XI) peut aussi être obtenu par action du trichloroacétonitrile en présence d'une base minérale ou organique sur un produit de formule générale :

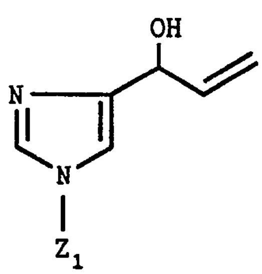

(XIV)

dans laquelle $Z_1$ est défini comme précédemment.

Il est particulièrement avantageux d'utiliser comme base le diaza-1,8 bicyclo[5,4,0] undécène-7 (DBU) en opérant dans un solvant organique inerte tel que le chlorure de méthylène à une température voisine de 20 °C. Cependant de bons résultats sont également obtenus en utilisant comme base un hydrure de métal alcalin (hydrure de sodium), un dérivé organométallique (butyllithium) ou une base organique tertiaire telle que la triéthylamine ou la diméthylaminopyridine.

Le produit de formule générale (XIV) peut être obtenu par action du bromure ou du chlorure de vinylmagnésium sur l'aldéhyde de formule générale :

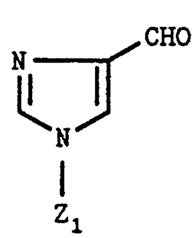

(XV)

dans laquelle $Z_1$ est défini comme précédemment, suivie de l'hydrolyse de l'alcoolate de magnésium obtenu.

Le produit de formule générale (XV) peut être obtenu selon le procédé décrit par J.M. Kokasa et al., J. Org. Chem., 48, 3605 (1983).

Le produit de formule générale (VII) peut aussi être obtenu par action de la N-chlorosuccinimide en présence d'eau sur le produit de formule générale :

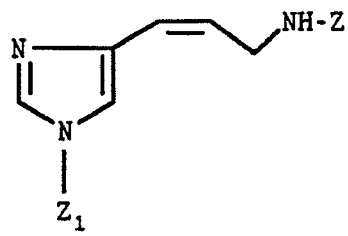

(XVI)

dans lesquelles Z et $Z_1$ sont définis comme précédemment.

Généralement on opère dans un solvant organique tel qu'un éther (éther diméthylique de l'éthylèneglycol) à une température voisine de 20 °C.

On obtient à côté du produit attendu de formule générale (VII) un mélange des chlorhydrines de formule générale (VI) et (VIII) qui peut être oxydé en produit de formule générale (VII) dans les conditions décrites précédemment pour préparer le produit de formule générale (VII) à partir du produit de formule générale (VIII).

Le produit de formule générale (XVI) peut être obtenu selon les méthodes habituelles d'introduction d'un groupement protecteur Z à partir d'un produit de formule générale :

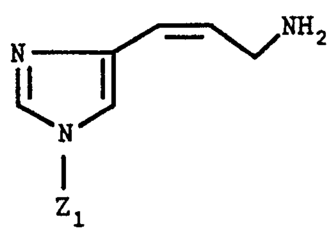

(XVII)

dans laquelle $Z_1$ est défini comme précédemment, sur lequel on fait réagir un réactif de blocage de la fonction amine.

Pour introduire comme groupement protecteur le groupement tert.butoxycarbonyle, il est particulièrement avantageux de faire réagir le di-tert.butyldicarbonate dans un solvant organique inerte tel que le chlorure de méthylène.

Le produit de formule générale (XVII) peut être obtenu par hydrolyse alcaline du produit de formule générale :

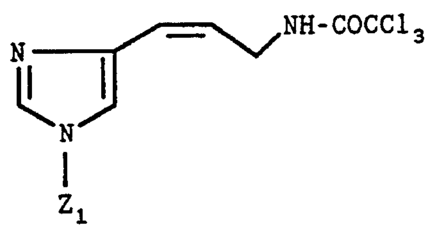

(XVIII)

dans laquelle $Z_1$ est défini comme précédemment.

Le produit de formule générale (XVIII) peut être obtenu par action du trichloroacétamide sodé ou du N-triméthylsilyle trichloroacétamide sodé et du bromure de triphénylvinylphosphonium sur l'aldéhyde de formule générale (XV).

Le produit de formule générale (VIII) peut aussi être obtenu par action de la N-chlorosuccinimide en

présence d'eau sur le produit de formule générale :

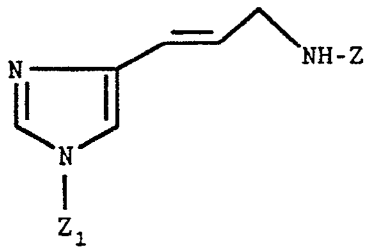

(XIX)

dans laquelle Z et $Z_1$ sont définis comme précédemment.

On opère généralement dans un solvant organique tel qu'un éther (éther diméthylique de l'éthylèneglycol) à une température voisine de 20 °C.

Le produit de formule générale (XIX) peut être obtenu à partir du produit de formule générale (XIII) sur lequel on fait réagir un réactif de blocage de la fonction amine.

Généralement, on opère dans les conditions décrites précédemment pour préparer le produit de formule générale (XVI) à partir du produit de formule générale (XVIII).

Selon l'invention, le racémique thréo du produit de formule (I) peut aussi être obtenu par réduction au moyen d'acide formique en présence de zinc d'un dérivé azo de formule générale :

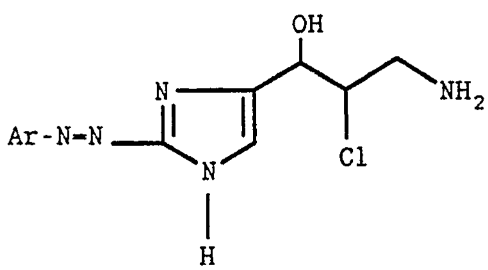

(XX)

dans laquelle Ar représente un radical phényle éventuellement substitué par un ou plusieurs radicaux, identiques ou différents, choisis parmi les atomes d'halogène (fluor, chlore, brome) et les radicaux alcoyles contenant 1 à 4 atomes de carbone ou alkoxycarbonyles dont la partie alcoyle contient 1 à 4 atomes de carbone ou nitro.

Généralement, la réduction du produit de formule générale (XX) est effectuée en opérant dans un solvant organique choisi parmi les alcools (méthanol, éthanol, isopropanol), les acides organiques (acide acétique, acide formique) et les solvants chlorés (chlorure de méthylène, dichloro-1,2 éthane) ou dans un mélange de ces solvants. Il est particulièrement avantageux d'opérer dans un acide organique (acide acétique) éventuellement en mélange avec un alcool (méthanol, éthanol, isopropanol) ou dans un mélange d'acide formique avec un solvant chloré. Généralement, la réduction s'effectue à une température comprise entre 0 et 25 °C. Dans le but d'accélérer la réaction, il peut être avantageux d'opérer en présence d'ultrasons.

Le produit de formule générale (XX) peut être obtenu à partir d'un produit de formule générale (II) par toute méthode permettant de remplacer le groupement protecteur Z de la fonction amine par un atome d'hydrogène sans toucher au reste de la molécule.

Généralement, le remplacement du groupement protecteur Z par un atome d'hydrogène s'effectue en milieu acide en opérant dans l'eau ou un alcool à une température comprise de préférence entre 0 et 50 °C. Comme acides peuvent être utilisés des acides minéraux tels que les acides chlorhydrique ou sulfurique ou des acides organiques tels que l'acide formique. Comme alcools peuvent être utilisés le méthanol, l'éthanol, le propanol ou l'isopropanol.

Le racémique thréo du produit de formule (I) peut se présenter sous forme de sels d'addition avec les acides minéraux ou organiques tels que l'acide chlorhydrique.

Le racémique thréo du produit de formule (I), éventuellement sous forme de sel, peut être séparé en ses deux énantiomères par chromatographie sur une colonne possédant une phase stationnaire chirale.

Le racémique thréo du produit de formule (I), éventuellement sous forme de sel, présente les propriétés antitumorales remarquables de la girolline.

Le racémique thréo du produit de formule (I) présente l'avantage d'être plus facilement accessible que la girolline elle-même sans pour autant présenter des inconvénients qui pourraient nuire à son utilisation en thérapeutique humaine en tant que médicament.

La présente invention concerne également les compositions pharmaceutiques qui contiennent le racémique thréo du produit de formule (I) en association avec tout autre produit pharmaceutiquement acceptable, qu'il soit inerte ou physiologiquement actif.

Ces compositions peuvent être présentées sous toute forme appropriée à la voie d'administration prévue. La voie parentérale est la voie d'administration préférentielle et notamment la voie intra-veineuse.

Les compositions selon l'invention pour administration parentérale peuvent être des solutions stériles aqueuses ou non aqueuses, des suspensions ou des émulsions. Comme solvant ou véhicule, on peut employer le propylèneglycol, les huiles végétales, en particulier l'huile d'olive, et les esters organiques injectables, par exemple l'oléate d'éthyle. Ces compositions peuvent également comprendre des adjuvants en particulier des agents mouillants, émulsifiants ou dispersants. La stérilisation peut se faire de plusieurs façons, par exemple à l'aide d'un filtre bactériologique, en incorporant à la composition des agents stérilisants, par irradiation ou par chauffage. Elles peuvent également être préparées sous forme de compositions solides stériles qui peuvent être dissoutes ou dispersées au moment de l'emploi dans de l'eau stérile ou tout autre milieu stérile injectable.

Le racémique thréo du produit de formule (I) est particulièrement utile dans le traitement des hémopathies malignes et des tumeurs solides à des doses journalières généralement comprises entre 0,1 et 1 mg/kg par voie intraveineuse pour un adulte.

Les exemples suivants, donnés à titre non limitatif, montrent comment l'invention peut être mise en pratique.

## EXEMPLE 1

Une suspension de 0,92 g d'oxyde de platine dans 100 cm3 de méthanol est agitée sous courant d'hydrogène à température ambiante et à pression atmosphérique pendant 45 minutes. La suspension de platine préréduit ainsi obtenue est diluée par 600 cm3 de méthanol.On ajoute alors 44,4 cm3 d'une solution méthanolique d'acide chlorhydrique 1N puis 9,2g de tert-butoxycarbonylamino-3 [(chloro-4 phénylazo)-2 1H-imidazolyl-4]-1 chloro-2 propanol-1-(1RS,2RS). Le milieu réactionnel est agité sous courant d'hydrogène, à pression atmosphérique et à température ambiante pendant 72 heures. Après avoir purgé l'appareillage à l'azote le platine est séparé par filtration. La solution méthanolique obtenue est concentrée à sec sous pression réduite (20 mm de mercure; 2,7 kPa) à 40 °C. L'huile récupérée (10,5 g) est reprise par 200 cm3 de méthanol et par 250 cm3 d'une solution méthanolique d'acide chlorhydrique 3N. La solution obtenue est agitée à température ambiante pendant 22 heures puis concentrée à sec sous pression réduite (20 mm de mercure; 2,7 kPa) à 40 °C. Le résidu huileux obtenu est dissous dans 500 cm3 d'un mélange d'acétate d'éthyle-méthyléthylcétone-acide formique-eau (5-3-1-1 en volumes) et de 50 cm3 de méthanol. La solution ainsi obtenue est versée sur 1 kg de silice (Merck, 0,040-0,063 mm) imprégnée d'acétate d'éthyle et contenue dans une colonne de 8,5 cm de diamètre. On élue tout d'abord avec 12 litres d'un mélange d'acétate d'éthyle-méthyléthylcétone-acide formique-eau (5-3-0,5-0,5 en volumes) puis on fait croître les proportions d'acide formique et d'eau en éluant avec 3 litres d'un mélange quaternaire (5-3-0,75-0,75 en volumes) et enfin avec 12 litres d'un mélange quaternaire (5-3-1-1 en volumes). Les 6 derniers litres d'éluat récupérés sont concentrés à sec sous pression réduite (20 mm de mercure; 2,7 kPa) à 45 °C. On récupère 4,32 g de résidu sous forme de meringue qui est mis en solution dans 100 cm3 de méthanol et filtré sur une colonne de 2,5 cm de diamètre contenant 190 g de gel de Sephadex LH 20 en éluant avec du méthanol. Après concentration de la solution méthanolique à sec sous pression réduite (1 mm de mercure ; 0,13 kPa) à 40 °C, on obtient 3,19 g de dichlorhydrate d'amino-3 (amino-2 1H-imidazolyl-4)-1 chloro-2 propanol-1-(1RS,2RS) sous forme d'une huile visqueuse brun clair dont le spectre RMN du proton dans le diméthylsulfoxyde deutérié présente les caractéristiques suivantes :

$-CH_2-NH_2$ $\delta$ = 3,1 et 3,3 ppm

$-\underline{CH}(Cl)-$ $\delta$ = 4,55 ppm

$-\underline{CH}(OH)-$ $\delta$ = 4,95 ppm

$=\underline{CH}-(5)$ $\delta$ = 6,75 ppm

## EXEMPLE 2

On solubilise 12,2 g de tert-butoxycarbonylamino-3 chloro-2 (1H-imidazolyl-4)-1 propanol-1-(1RS,2RS) dans 100 cm3 de méthanol. On ajoute ensuite à cette solution refroidie à 2 °C 425 cm3 d'eau puis 365 cm3 d'une solution aqueuse à 10% de carbonate de sodium. On ajoute alors en 20 minutes et en maintenant la température du milieu réactionnel à 2 °C une solution de chlorure de chloro-4 phényldiazonium (obtenue à partir de 5,65 g de chloro-4 aniline dissoute dans 330 cm3 d'acide chlorhydrique 1N à laquelle on ajoute à 2 °C, en 5 minutes et sous agitation, une solution de 3,12 g de nitrite de sodium dans 55 cm3 d'eau). Après 20 minutes d'agitation supplémentaire à 2 °C on ajoute 450 cm3 de chloroforme puis on laisse décanter. La phase aqueuse est extraite deux fois par 400 cm3 de chloroforme puis les phases organiques chloroformiques sont réunies, séchées sur sulfate de magnésium, filtrées puis concentrées à sec sous pression réduite (20 mm de mercure; 2,7 kPa) à 40 °C. Le résidu rouge (17,7 g) est alors dissous dans 400 cm3 de chloroforme contenant 4 cm3 de méthanol et la solution obtenue est versée sur 550 g de silice (Merck, 0,040-0,063 mm) imprégnée de chloroforme et contenue dans une colonne de 6,4 cm de diamètre. On élue avec 20 litres d'un mélange de chloroforme et de méthanol (99,5-0,5 en volumes) puis avec 3,75 litres d'un mélange de chloroforme et de méthanol (99-1 en volumes). Ces éluats sont éliminés. On élue enfin avec 2,5 litres d'un mélange de chloroforme et de méthanol (96-4 en volumes) et l'éluat correspondant est concentré à sec sous pression réduite (20 mm de mercure; 2,7 kPa) à 40 °C. On obtient 9,3 g de tert-butoxycarbonylamino-3 chloro-2 [(chloro-4 phénylazo)-2 1H-imidazolyl-4]-1 propanol-1-(1RS,2RS) sous forme d'un solide jaune fondant à 194 °C et dont le spectre RMN du proton dans le diméthylsulfoxyde deutérié présente les caractéristiques suivantes:

-C($CH_3$)$_3$ $\delta$ = 1,43 ppm
-$\underline{CH_2}$-NH- $\delta$ = 3,40 ppm
-$\underline{CH}$(Cl)- $\delta$ = 4,45 ppm
-$\underline{CH}$(OH)- $\delta$ = 4,95 ppm
=$\underline{CH}$-(5) $\delta$ = 7,33 ppm

EXEMPLE 3

A 20 g de tert-butoxycarbonylamino-3 chloro-2 (trityl-1 1H-imidazolyl-4)-1 propanol-1-(1RS,2RS) dans 600 cm3 de propanol-1 on ajoute 2,2 cm3 d'acide acétique glacial. La solution obtenue est portée au reflux sous atmosphère d'azote pendant 24 heures. Après refroidissement à température ambiante le milieu réactionnel est concentré à sec sous pression réduite (20 mm de mercure ; 2,7 kPa) à 40 °C. Le résidu obtenu est dissous dans 50 cm3 de chlorure de méthylène et la solution versée sur 500 g de silice (Merck, 0,063-0,200 mm) contenue dans une colonne de 6 cm de diamètre. On élue avec 2 litres d'un mélange de chlorure de méthylène et de méthanol (95-5 en volumes). Ces éluats sont éliminés puis on poursuit avec 4 litres d'un mélange de chlorure de méthylène et de méthanol (9-1 en volumes). Ces éluats sont concentrés à sec sous pression réduite (20 mm de mercure ; 2,7 kPa) à 40 °C. On obtient ainsi 8,95 g de tert-butoxycarbonylamino-3 chloro-2 (1H-imidazolyl-4)-1 propanol-1-(1RS,2RS) sous forme de meringue dont le spectre RMN dans le chloroforme deutérié présente les caractéristiques suivantes :

-C($CH_3$)$_3$ $\delta$ = 1,43 ppm
-$\underline{CH_2}$-NH- $\delta$ = 3,30 et 3,63 ppm
-$\underline{CH}$(Cl)- $\delta$ = 4,30 ppm
-$\underline{CH}$(OH)- $\delta$ = 4,93 ppm
=$\underline{CH}$-(5) $\delta$ = 7,02 ppm
=$\underline{CH}$-(2) $\delta$ = 7,55 ppm

EXEMPLE 4

A 65,8 g de N-tert-butoxycarbonyl chloro-2 oxo-3 (trityl-1 1H-imidazolyl-4)-3 propanamine-1-(R,S) dans 650 cm3 de tétrahydrofuranne anhydre on ajoute sous agitation en 1 heure 30 minutes, à -75 °C et sous atmosphère d'azote, 255,3 cm3 de tri-sec-butylborohydrure de lithium. Après 0,5 heure d'agitation à -75 °C le milieu réactionnel est hydrolysé par 1 litre d'une solution saturée de chlorure d'ammonium en maintenant la température proche de -60 °C. On réchauffe la masse réactionnelle à température ambiante on ajoute alors 1500 cm3 de chlorure de méthylène puis 77 cm3 d'éthanolamine. On agite pendant 3 heures puis on décante le milieu réactionnel. La phase organique est lavée par 2 fois 600 cm3 d'eau. Après addition de 77 cm3 d'éthanolamine la solution obtenue est agitée pendant 2 heures à température ambiante puis lavée par 2 fois 600 cm3 d'eau. La solution organique obtenue est séchée sur sulfate de magnésium puis filtrée et

concentrée à sec sous pression réduite (20 mm de mercure ; 2,7 kPa) à 40° C. Le résidu obtenu est repris par 200 cm3 de chlorure de méthylène et la solution obtenue est versée sur 1 kg de silice contenue dans une colonne de 8 cm de diamètre. On élue avec un mélange de chlorure de méthylène et d'acétate d'éthyle (8-2 en volumes). Les 6 premiers litres sont éliminés et les 3,8 litres suivants sont évaporés à sec sous pression réduite (20 mm de mercure ; 2,7 kPa) à 40° C. on obtient ainsi 49 g de tert-butoxycarbonylamino-3 chloro-2 (trityl-1 1H-imidazolyl-4)-1 propanol-1-(1RS,2RS) sous forme d'un solide blanc fondant à 132° C et dont le spectre RMN du proton dans le chloroforme deutérié présente les caractéristiques suivantes :

$-C(CH_3)_3$ δ = 1,50 ppm
$-CH_2-NH-$ δ = 3,40 et 3,75 ppm
$-CH(Cl)-$ δ = 4,37 ppm
$-CH(OH)-$ δ = 5,00 ppm
$=CH-(5)$ δ = 6,94 ppm
$=CH-(2)$ δ = 7,45 ppm

EXEMPLE 5

A 84,4 g de tert-butoxycarbonylamino-3 chloro-2 (trityl-1 1H-imidazolyl-4)-1 propanol-1-(1RS,2SR) dans 1500 cm3 de chlorure de méthylène on ajoute 170 g de dioxyde de manganèse activé. Le mélange est agité au reflux pendant 5 heures. Le milieu réactionnel est refroidi à température voisine de 20° C puis les sels de manganèse sont filtrés et lavés par 4 fois 400 cm3 de chlorure de méthylène. Les phases organiques réunies sont concentrées à sec sous pression réduite (20 mm de mercure ; 2,7 kPa) à 40° C. Le résidu est solubilisé dans 400 cm3 de chlorure de méthylène et la solution versée sur 1600 g de silice (Merck, 0,040-0,063 mm). On élue avec un mélange de chlorure de méthylène et d'acétate d'éthyle (95-5 en volumes). Les 3500 premiers cm3 sont éliminés et les 3250 cm3 suivants qui contiennent le produit sont concentrés sous pression réduite (20 mm de mercure ; 2,7 kPa) à 40° C. On obtient ainsi 71,5 g de N-tert-butoxycarbonyl-1 chloro-2 oxo-3 (trityl-1 1H-imidazolyl-4)-3 propanamine-1-(R,S) sous forme de meringue dont le spectre RMN du proton dans le chloroforme deutérié présente les caractéristiques suivantes :

$-C(CH_3)3$ δ = 1,43 ppm
$-CH_2-NH-$ δ = 3,77 ppm
$-CH(Cl)-$ δ = 5,48 ppm
$=CH-(2)$ δ = 7,50 ppm
$=CH-(5)$ δ = 7,70 ppm

EXEMPLE 6

A 45,4 g d'amino-3 chloro-2 (trityl-1 1H-imidazolyl-4)-1 propanol-1-(1RS,2SR) on ajoute sous atmosphère d'azote à température voisine de 20° C et sous agitation 800 cm3 de chlorure de méthylène puis 24,5 g de di-tert-butyldicarbonate. On laisse les réactifs ainsi en contact pendant 1 heure puis on ajoute 8 g de diéthylamine. Le milieu réactionnel est alors maintenu sous agitation pendant 15 minutes puis on lave la phase chlorométhylènique par 4 fois 300 cm3 d'eau, la sèche sur sulfate de magnésium, la filtre puis la concentre à sec sous pression réduite (20 mm de mercure ; 2,7 kPa) à 40° C. Le résidu obtenu est dissous dans 200 cm3 de chlorure de méthylène et la solution versée sur 370 g de silice (Merck, 0,063-0,200 mm) contenue dans une colonne de 4 cm de diamètre. On élue avec un mélange de chlorure de méthylène et de méthanol (98-2 en volumes). Les premiers 1500 cm3 sont éliminés et les 3500 cm3 suivants sont évaporés à sec sous pression réduite (20 mm de mercure ; 2,7 kPa) à 45° C. On récupère ainsi 45 g de tert-butoxycarbonylamino-3 chloro-2 (trityl-1 1H-imidazolyl-4)-1 propanol-1-(1RS,2SR) sous forme d'un solide blanc fondant à 168° C et dont le spectre RMN du proton dans le chloroforme présente les caractéristiques suivantes :

$-C(CH_3)3$ δ = 1,46 ppm
$-CH_2-NH-$ δ = 3,50 et 3,86 ppm
$-CH(Cl)-$ δ = 4,32 ppm
$-CH(OH)-$ δ = 4,68 ppm
$=CH-(5)$ δ = 6,85 ppm
$=CH-(2)$ δ = 7,41 ppm

EXEMPLE 7

A 48,4 g de chloro-5 trichlorométhyl-2 (trityl-1 1H-imidazolyl-4)-6 4H-dihydro-5,6 oxazine-1,3-(5RS,6SR) solubilisés dans 604 cm3 de tétrahydrofuranne et 1208 cm3 d'éther isopropylique on ajoute en 5 minutes sous agitation et en maintenant la température voisine de 20°C 1208 cm3 d'acide chlorhydrique 6N. On agite encore 5 minutes à 20°C puis on décante et lave la phase organique par 2 fois 200 cm3 d'eau. Les phases aqueuses réunies sont extraites par 200 cm3 d'oxyde d'isopropyle puis refroidies à une température comprise entre 0°C et 5°C. On ajoute alors sous agitation, progressivement et tout en maintenant la température inférieure à 5°C, 1500 cm3 de soude 5N ce qui conduit à un pH voisin de 9. Après 8 minutes d'agitation dans ces conditions on extrait le milieu réactionnel par 1 litre de chlorure de méthylène puis encore par 2 fois 500 cm3 de ce même solvant. Les phases organiques réunies sont séchées sur sulfate de magnésium, filtrées puis concentrées à sec sous pression réduite (20 mm de mercure ; 2,7 kPa) à 40°C. On obtient ainsi 32,6 g de amino-3 chloro-2 (trityl-1 1H-imidazolyl-4)-1 propanol-1-(1RS,2SR) sous forme d'une huile brun clair dont le spectre RMN du proton dans le chloroforme deutérié présente les caractéristiques suivantes :

$-CH_2-NH_2$ $\delta$ = 3,18 ppm
$-\overline{CH}(Cl)-$ $\delta$ = 4,32 ppm
$-\overline{CH}(OH)-$ $\delta$ = 4,90 ppm
$=\overline{CH}-(5)$ $\delta$ = 6,90 ppm
$=\overline{CH}-(2)$ $\delta$ = 7,44 ppm

EXEMPLE 8

A 76,6 g de N-trichloroacétyl (trityl-1 1H-imidazolyl-4)-3 propène-2 amine-1-(E) solubilisés dans 920 cm3 de chlorure de méthylène on ajoute 920 cm3 d'eau. On agite vigoureusement tout en faisant barboter dans le milieu réactionnel du dioxyde de carbone pendant 15 minutes à un débit de 0,6 litre par minute. On refroidit le milieu réactionnel à 10°C puis on ajoute progressivement en 1 heure, tout en maintenant le débit gazeux à 0,6 litre par minute et la température inférieure à 10°C, 19 g d'hypochlorite de calcium à 70 % finement broyé. A la fin de l'introduction de l'hypochlorite on laisse encore sous agitation et sous courant de dioxyde de carbone pendant 0,5 heure. Le mélange réactionnel est alors filtré et le fitrat décanté. La phase organique est séparée et agitée en présence de 250 cm3 d'une solution aqueuse de thiosulfate de sodium à 10 % pendant 10 minutes. La solution chlorométhylènique obtenue après décantation est lavée par 250 cm3 d'eau puis séchée sur sulfate de magnésium, filtrée et concentrée à sec sous pression réduite (20 mm de mercure ; 2,7 kPa) à 40°C.Le résidu est repris par 200 cm3 de chlorure de méthylène et la solution versée sur 450 g de silice (Merck, 0,063-0,200 mm) contenue dans une colonne de 5,2 cm de diamètre. On élue avec du chlorure de méthylène. Les 900 premiers cm3 d'éluats sont éliminés puis les 7,4 litres suivants sont concentrés à sec sous pression réduite (20 mm de mercure; 2,7 kPa) à 40°C. On obtient 39,6g de chloro-5 trichlorométhyl-2 (trityl-1 1H-imidazolyl-4)-6 4H-dihydro-5,6 oxazine-1,3-(5RS,6SR) sous forme d'un solide blanc fondant à 168°C et dont le spectre du proton dans le chloroforme deutérié présente les caractéristiques suivantes :

$-CH_2-N=$ $\delta$ = 3,82 et 3,90 ppm
$-\overline{CH}(Cl)-$ $\delta$ = 4,73 ppm
$=\overline{CH}-O-$ $\delta$ = 5,53 ppm
$=\overline{CH}-(5)$ $\delta$ = 7,00 ppm
$=\overline{CH}-(2)$ $\delta$ = 7,45 ppm

EXEMPLE 9

A 150 g de (phtalimido-3 propène-1 yl-1)-4 trityl-1 1H-imidazole-(E) dans 1500 cm3 de toluène on ajoute, à une température proche de 20°C, 241 cm3 de méthylhydrazine. Le mélange réactionnel est chauffé au reflux et sous atmosphère d'azote pendant 3 heures. On refroidit jusqu'à une température voisine de 40°C puis on ajoute en maintenant à cette température 750 cm3 d'eau. La phase toluènique est séparée par décantation puis lavée par 2 fois 500 cm3 d'eau. La solution organique obtenue qui contient 110 g de (trityl-1 1H-imidazolyl-4)-3 propène-2 amine-1-(E) est séchée sur sulfate de sodium, filtrée puis diluée par 1000 cm3 de chlorure de méthylène. On ajoute ensuite, sous courant d'azote et en 10 minutes, 69 cm3 d'hexachloroacétone. Une légère exothermie est observée au cours de l'addition qui fait passer la

température du milieu réactionnel de 20° C à 34° C. Après 1 heure d'agitation le mélange réactionnel est évaporé à sec sous pression réduite (20 mm de mercure ; 2,7 kPa) à 40° C et le résidu obtenu est repris par 200 cm3 d'oxyde d'isopropyle, trituré puis filtré. On obtient 121 g de N-trichloroacéthyl (trityl-1 1H-imidazolyl-4)-3 propène-2 amine-1-(E) sous forme d'un solide blanc fondant à 195° C et dont le spectre RMN du proton dans le chloroforme deutérié présente les caractéristiques suivantes :

$-CH_2-NH-$ $\delta$ = 4,10 ppm
$=\overline{CH}-CH_2-$ $\delta$ = 6,35 ppm
$-\overline{CH}=CH-CH_2-$ $\delta$ = 6,50 ppm
$=\overline{CH}-(5)$ $\delta$ = 6,80 ppm
$=\overline{CH}-(2)$ $\delta$ = 7,44 ppm

Le (phtalimido-3 propène-1 yl-1)-4 trityl-1 1H-imidazole-(E) peut être préparé suivant la méthode décrite par G. DE NANTEUIL et al., Bull. Soc. Chim. Fr., 813 (1986).

## EXEMPLE 10

A 66 g de (trityl-1 1H-imidazolyl-4)-1 propène-2 ol-1-(R,S) dans 660 cm3 de chlorure de méthylène on ajoute 27 g de diaza-1,8 bicyclo[5,4,0]undécène-7 puis 39 g de trichoracétonitrile. Le mélange réactionnel est agité à température voisine de 20° C pendant 24 heures. La solution brunâtre obtenue est versée directement sur 2 kg de silice (Merck, 0,063-0,200 mm) contenue dans une colonne de 8 cm de diamètre. On élue tout d'abord avec 3 litres d'un mélange de chlorure de méthylène et d'acétate d'éthyle (95-5 en volumes). Ces éluats sont éliminés puis on poursuit l'élution avec un mélange de chlorure de méthylène et d'acétate d'éthyle (90-10 en volumes). Les 2,5 premiers litres ne sont pas conservés puis les 3,5 litres suivants sont concentrés à sec sous pression réduite (20 mm de mercure ; 2,7 kPa) à 40° C. On obtient ainsi 17,5 g de N-trichloroacétyl (trityl-1 1H-imidazolyl-4)-3 propène-2 amine-1-(E).

## EXEMPLE 11

A 338 g de trityl-1 imidazolecarboxaldéhyde-4 dans 1800 cm3 de tétrahydrofuranne anhydre on ajoute progressivement en 3 heures sous agitation et sous atmosphère d'azote, en maintenant à une température voisine de -10° C, 1300 cm3 d'une solution 1M de bromure de vinylmagnésium dans le tétrahydrofuranne. Après 1 heure d'agitation à -10° C on hydrolyse le mélange réactionnel à cette même température avec 3000 cm3 d'une solution aqueuse de chlorure d'ammonium. La phase organique est décantée et la phase aqueuse est extraite par 2 fois 1000 cm3 de chloroforme. Les phases organiques réunies sont séchées sur sulfate de magnésium, filtrées et concentrées à sec sous pression réduite (20 mm de mercure ; 2,7 kPa) à 40° C. Le résidu obtenu est dissous dans 1 litre de chlorure de méthylène puis la solution obtenue est versée sur 4,5 kg de silice (Merck, 0,063-0,200 mm) contenue dans une colonne de 8 cm de diamètre. On élue avec 12 litres d'un mélange de chlorure de méthylène et d'acétate d'éthyle (90-10 en volumes). Ces éluats sont éliminés puis on poursuit la chromatographie avec 25 litres d'un mélange de chlorure de méthylène et de méthanol (94-4 en volumes). L'éluat est alors concentré à sec sous pression réduite (20 mm de mercure ; 2,7 kPa) à 45° C. On obtient 202 g de (trityl-1 1H-imidazolyl-4)-1 propène-2 ol-1-(R,S) sous forme d'un solide blanc fondant à 170° C et dont le spectre RMNdu proton dans le chloroforme deutérié présente les caractéristiques suivantes :

$-CH(OH)-$ $\delta$ = 5,17 ppm
$-\overline{CH}=CH_2$ $\delta$ = 5,17 et 5,36 ppm
$-CH=\overline{CH_2}$ $\delta$ = 6,10 ppm
$=\overline{CH}-(5)$ $\delta$ = 6,73 ppm
$=\overline{CH}-(2)$ $\delta$ = 7,42 ppm

Le trityl-1 1H-imidazolecarboxaldéhyde-4 peut être préparé selon J.M.KOKOSA et al., J. Org. Chem., 48, 3605 (1983).

## EXEMPLE 12

A une solution de 0,94 g de N-tert-butoxycarbonyl (trityl-1 1H-imidazolyl-4)-3 propène-2 amine-1-(Z) dans 10 cm3 d'éther diméthylique de l'éthylèneglycol on ajoute à température voisine de 20° C, 0,8 g de N-chlorosuccinimide puis 0,93 cm3 d'eau. Après 48 heures d'agitation on dilue le mélange réactionnel par

50 cm3 d'eau puis on extrait par 3 fois 25 cm3 d'acétate d'éthyle. Les phases organiques réunies sont séchées sur sulfate de magnesium, filtrées puis concentrées à sec sous pression réduite (20 mm de mercure ; 2,7 kPa) à 40 °C. Le résidu huileux est repris par 4 cm3 de cyclohexane puis la solution obtenue est versée sur 25 g de silice (Merck, 0,040-0,063 mm) contenue dans une colonne de 2,5 cm de diamètre. On élue avec un mélange de cyclohexane et d'acétate d'éthyle (80-20 en volumes). Les 80 premiers cm3 sont éliminés, les éluats correspondant aux 30 cm3 suivants sont concentrés à sec sous pression réduite (20 mm de mercure ; 2,7 kPa) à 40 °C. On récupère ainsi 0,29 g de N-tert-butoxycarbonyl chloro-2 oxo-3 (trityl-1 1H-imidazolyl-4)-3 propanamine-1-(R,S) présentant des caractéristiques identiques à celles du produit de l'exemple 5. Les éluats correspondant aux 300 cm3 suivants sont éliminés puis les 100 cm3 d'éluats qui suivent sont évaporés à sec sous pression réduite (20 mm de mercure ; 2,7 kPa) à 40 °C. On obtient alors 0,22 g de N-tert-butoxycarbonylamino-3 chloro-2 (trityl-1 1H-imidazolyl-4)-1 propanol-1 sous forme de mélange d'isomères. Ce mélanges d'isomères conduit par oxydation par le dioxyde de manganèse activé, dans les conditions décrites dans l'exemple 5, au N-tert-butoxycarbonyl chloro-2 oxo-3 (trityl-1 1H-imidazolyl-4)-3 propanamine-1-(R,S).

EXEMPLE 13

A 3,1 g de N-trichloroacétyl (trityl-1 1H-imidazolyl-4)-3 propène-2 amine-1-(Z) solubilisés dans 30 cm3 de dioxanne on ajoute à température proche de 20 °C et sous agitation 15 cm3 de soude 1N. Au bout de 5 heures on dilue par 100 cm3 d'eau puis on extrait par 3 fois 100 cm3 de chlorure de méthylène. Les phases organiques sont réunies, séchées sur sulfate de magnésium puis concentrées sous pression réduite (20 mm de mercure ; 2,7 kPa) à 40 °C. On récupère ainsi 2,5 g de (trityl-1 1H-imidazolyl-4)-3 propène-2 amine-1-(Z) utilisé tel quel dans l'étape suivante.

A 2,5 g de (trityl-1 1H-imidazolyl-4)-3 propène-2 amine-1-(Z) solubilisés dans 35 cm3 de chlorure de méthylène on ajoute, sous atmosphère d'azote et à une température voisine de 20 °C, 2 g de di-tert-butyldicarbonate. On agite le mélange réactionnel pendant 24 heures puis on ajoute 10 cm3 de soude 1N et on laisse en contact sous agitation pendant 15 minutes. La phase organique est ensuite décantée, séchée sur sulfate de magnésium, filtrée puis concentrée à sec sous pression réduite (20 mm de mercure ; 2,7 kPa) à 40 °C. Le résidu est repris par 10 cm3 de chlorure de méthylène puis la solution obtenue est versée sur 50 g de silice (Merck, 0,040-0,063 mm) contenue dans une colonne de 3 cm de diamètre. On élue au chlorure de méthylène. Les premiers 350 cm3 sont éliminés puis les 1500 cm3 suivants sont concentrés à sec sous pression réduite (20 mm de mercure; 2,7 kPa) à 40 °C. On obtient 2,05 g de N-tert-butoxycarbonyl (trityl-1 1H-imidazolyl-4)-3 propène-2 amine-1-(Z) sous forme d'un solide blanc fondant à 156 °C et dont le spectre RMN du proton dans le chloroforme deutérié présente les caractéristiques suivantes :

$-C(\underline{CH_3})_3$ $\delta = 1,45$ ppm
$-\underline{CH_2}-NH-$ $\delta = 4,13$ ppm
$=\underline{CH}-CH_2-$ $\delta = 5,65$ ppm
$-\underline{CH}=CH-CH_2-$ $\delta = 6,27$ ppm
$=\underline{CH}-(5)$ $\delta = 6,77$ ppm
$=\underline{CH}-(2)$ $\delta = 7,44$ ppm

EXEMPLE 14

A 50 cm3 de tétrahydrofuranne anhydre on ajoute, sous atmosphère d'azote, 2,35 g d'hydrure de sodium à 55 % dans l'huile de vaseline. On ajoute ensuite en 10 minutes, à température voisine de 20 °C, 7,9 g de trichloracétamide en solution dans 100 cm3 de tétrahydrofuranne anhydre. On laisse agiter 15 minutes puis on ajoute 10,14 g de trityl-1 1H-imidazolecarboxaldéhyde-4 en solution dans 100 cm3 de tétrahydrofuranne anhydre puis 18,8 g de bromure de triphénylvinylphosphonium et enfin 250 cm3 de tétrahydrofuranne anhydre. On agite le mélange réactionnel pendant 18 heures à température voisine de 20 °C. On hydrolyse avec 200 cm3 d'une solution aqueuse saturée de chlorure d'ammonium. La phase organique est décantée et séparée. La phase aqueuse est extraite par 3 fois 100 cm3 de chlorure de méthylène. Les solutions organiques sont toutes réunies, séchées sur sulfate de magnésium, filtrées puis concentrées à sec sous pression réduite (20 mm de mercure ; 2,7 kPa) à 40 °C. Le résidu est repris par 100 cm3 de chlorure de méthylène et la solution obtenue versée sur 350 g de silice (Merck, 0,063-0,200 mm) contenue dans une colonne de 4,5 cm de diamètre. On élue au chlorure de méthylène. Les 700

premiers cm3 sont éliminés puis les 900 cm3 suivants sont concentrés à sec sous pression réduite (20 mm de mercure ; 2,7 kPa) à 40 °C. On récupère 9,33 g de N-trichloroacétyl (trityl-1 1H-imidazolyl-4)-3 propène-2 amine-1-(Z) sous forme d'un solide blanc fondant à 170 °C et dont le spectre RMN du proton dans le chloroforme deutérié présente les caractéristiques suivantes :

$-CH_2-NH-$ δ = 4,23 ppm
$=CH-CH_2-$ δ = 5,78 ppm
$-CH=CH-CH_2-$ δ = 6,35 ppm
$=CH-(5)$ δ = 6,83 ppm
$=CH-(2)$ δ = 7,45 ppm

EXEMPLE 15

A une solution de 0,46 g de N-tert-butoxycarbonyl (trityl-1 1H-imidazolyl-4)-3 propène-2 amine-1-(E) dans un mélange de 18 cm3 de diméthoxy-1,2 éthane et de 2 cm3 d'eau on ajoute 0,27 g de N-chlorosuccinimide. Après 5 jours d'agitation à température proche de 20 °C on ajoute au milieu réactionnel 20 cm3 d'une solution à 10% de thiosulfate de sodium. On agite 10 minutes à une température voisine de 20 °C puis on extrait avec 3 fois 20 cm3 d'acétate d'éthyle. Les phases organiques sont réunies, séchées sur sulfate de magnésium et concentrées à sec sous pression réduite (20 mm de mercure; 2,7 kPa) à 40 °C. Le résidu obtenu est dissous dans 2 cm3 de chlorure de méthylène puis la solution obtenue est versée sur 15 g de silice (Merck, 0,04-0,063 mm) contenue dans une colonne de 1,2 cm de diamètre. On élue sous une pression de 40 kPa avec un mélange de chlorure de méthylène et de méthanol (9-1 en volumes). Les premiers 40 cm3 sont éliminés puis on récupère le produit attendu dans les 20 cm3 suivants. Ces éluats sont concentrés à sec sous pression réduite (20 mm de mercure ; 2,7 kPa) à 40 °C. On obtient 0,1 g de N-tert-butoxycarbonylamino-3 chloro-2 (trityl-1 1H-imidazolyl-4)-1 propanol-1-(1RS,2SR) sous forme d'un solide présentant les mêmes caractéristiques physiques et analytiques que le produit décrit dans l'exemple 6.

EXEMPLE 16

A 15 g de (phtalimido-3 propène-1 yl-1)-4 trityl-1 1H-imidazole-(E) dans 150 cm3 de toluène on ajoute, à une température voisine de 20 °C, 24,1 cm3 de méthylhydrazine. Le mélange réactionnel est chauffé au reflux sous atmosphère d'azote pendant 3 heures. On refroidit à une température voisine de 40 °C puis on ajoute, en maintenant le milieu réactionnel à cette température, 75 cm3 d'eau. La solution toluènique est séparée par décantation puis lavée par 2 fois 50 cm3 d'eau. A la solution organique obtenue, séchée sur sulfate de magnésium, on ajoute, à une température voisine de 20 °C, 18,2 g de di-tert-butyldicarbonate. On laisse sous agitation pendant 16 heures puis on concentre à sec le milieu réactionnel sous pression réduite (20 mm de mercure; 2,7 kPa) à 40 °C. Le résidu est ensuite solubilisé dans 60 cm3 de chlorure de méthylène et la solution obtenue versée sur 800 g de silice (Merck, 0,04-0,063 mm) contenue dans une colonne de 5 cm de diamètre. On élue sous une pression de 40 kPa avec un mélange de chlorure de méthylène et de méthanol (99-1 en volumes). Les premiers 500 cm3 sont éliminés puis on poursuit la chromatographie avec 1 litre d'un mélange de chlorure de méthylène et de méthanol (97-3 en volumes). Ces éluats sont concentrés à sec sous pression réduite (20 mm de mercure ; 2,7 kPa) à 40 °C. On obtient ainsi 6,4 g de N-tert-butoxycarbonyl (trityl-1 1H-imidazolyl-4)-3 propène-2 amine-1-(E) sous forme d'une meringue blanche dont le spectre RMN du proton dans le chloroforme deutérié présente les caractéristiques suivantes :

$-C(CH_3)_3$ δ = 1,45 ppm
$-CH_2-NH-$ δ = 3,87 ppm
$-CH=CH-CH_2-$ δ = 6,35 ppm
$=CH-(5)$ δ = 6,75 ppm
$=CH-(2)$ δ = 7,42 ppm

EXEMPLE 17

A l'aide de 2 colonnes chirales CROWNPAX CR(+) de 15 cm de longueur et de 4,6 cm de diamètre intérieur montées en série et thermostatées, ainsi que le flacon contenant la phase mobile, dans un bain

réfrigérant à +5° C, on sépare les 2 énantiomères en opérant sous 130 bars (13 MPa), à un débit de 0,4 cm3/minute et en suivant l'élution par détection UV à 220 nm. On effectue 200 injections de 20 µl d'une solution aqueuse d'acide perchlorique (pH 1,5) contenant 5,3 mg/cm3 d'amino-3 (amino-2 1H-imidazolyl-4)-1 chloro-2 propanol-1-(1RS,2RS). Dans ces conditions les temps de rétention des 2 énantiomères sont respectivement de 16,0 minutes et de 18,2 minutes. L'énantiomère correspondant au produit naturel extrait de Pseudaxinyssa cantharella tel qu'il est défini dans le brevet FR 2 585 020 est le second dans l'ordre d'élution. On récupère ainsi l'énantiomère naturel dans 215 cm3 de solution aqueuse d'acide perchlorique utilisée comme phase mobile. Cette solution est versée sur 1 litre de résine IRA 400 (Cl-) contenue dans une colonne de 4 cm de diamètre. On élue lentement avec de l'eau. Les 250 premiers cm3 sont éliminés puis les 625 cm3 suivants sont concentrés à sec sous pression réduite (1 mm de mercure ; 0,13 kPa) à 45° C. Le résidu obtenu est purifié en le solubilisant dans 0,5 cm3 de méthanol et en le filtrant sur 30 g de gel de Sephadex LH 20 contenus dans une colonne de 1,5 cm de diamètre. On élue au méthanol et les 62 premiers cm3 sont éliminés. Les 22 cm3 suivants sont concentrés à sec sous pression réduite (1 mm de mercure ; 0,13 kPa) à 30° C. On obtient ainsi 1,1 mg de dichlorhydrate d'amino-3 (amino-2 1H-imidazolyl-4)-1 chloro-2 propanol-1 énantiomériquement pur possédant un temps de rétention sur colonne CROWNPAK CR(+) identique à celui du produit naturel et dont le spectre RMN du proton dans le diméthylsulfoxyde deutérié présente les caractéritiques suivantes :

$-CH_2-NH_2$ δ = 3,1 et 3,3 ppm

$-\overline{CH}(Cl)-$ δ = 4,55 ppm

$-\overline{CH}(OH)-$ δ = 4,95 ppm

$=\overline{CH}-(5)$ δ = 6,75 ppm

## EXEMPLE 18

On dissout 15 g de dichlorhydrate d'amino-3 chloro-2 [(chloro-4 phénylazo)-2 1H-imidazolyl-4]-1 propanol-1-(1RS,2RS) dans 300 cm3 d'acide formique.

On ajoute alors, par petites fractions, 45 g de zinc en poudre. La suspension est agitée pendant 4 heures à une température voisine de 20° C. Le zinc est séparé par filtration et l'acide formique est distillé à 40° C sous pression réduite (15 mm de mercure ; 2,0 kPa). Le résidu huileux obtenu est dissous dans 100 cm3 d'éthanol. Les sels de zinc insolubles sont séparés par filtration et l'éthanol est distillé sous pression réduite (15 mm de mercure ; 2,0 kPa).

On obtient un résidu huileux (20 g) qui est purifié par chromatographie sur silice en éluant avec un mélange acétate d'éthyle-méthyléthylcétone-acide formique-eau dans les conditions décrites dans l'exemple 1. On obtient ainsi 6 g de dichlorhydrate d'amino-3 (amino-2 1H-imidazolyl-4)-1 chloro-2 propanol-1-(1RS,2RS) sous forme d'une huile dont les caractéristiques sont identiques à celles du produit de l'exemple 1.

Le chlorhydrate d'amino-3 chloro-2 [(chloro-4 phénylazo)-2 1H-imidazolyl-4)-1 propanol-1-(1RS,2RS) peut être préparé de la manière suivante :

On ajoute, à une température voisine de 20° C, 200 cm3 de méthanol chlorhydrique 3,65N à une suspension de 30 g de tert-butoxycarbonylamino-3 chloro-2 [(chloro-4 phénylazo)-2 1H-imidazolyl-4]-1 propanol-1-(1RS,2RS) dans 150 cm3 de méthanol. La solution obtenue est agitée pendant 1 heure à une température voisine de 20° C. Le produit cristallise lentement. A la suspension obtenue, on ajoute, goutte à goutte, 350 cm3 d'éther éthylique. Les cristaux jaunes sont séparés par filtration puis lavés par 20 cm3 d'éther éthylique et enfin séchés sous pression réduite (10 mm de mercure ; 1,3 kPa).

On obtient ainsi, avec un rendement de 89 %, 25 g de dichlorhydrate d'amino-3 chloro-2 [(chloro-4 phénylazo)-2 1H-imidazolyl-4]-1 propanol-1-(1RS,2RS) sous forme de cristaux jaunes fondant à 186° C.

## EXEMPLE 19

On prépare une solution contenant 20 mg/cm3 d'amino-3 (amino-2 1H-imidazolyl-4)-1 chloro-2 propanol-1- (1RS,2RS) en dissolvant 27,75 g du dichlorhydrate obtenu à l'exemple 1 dans du soluté physiologique apyrogène en quantité suffisante pour obtenir 100 cm3. La solution obtenue est répartie aseptiquement en ampoules à raison de 5 cm3 par ampoule. Les ampoules sont scellées ; elles contiennent chacune 100 mg d'amino-3 (amino-2 1H-imidazolyl-4)-1 chloro-2 propanol-1-(1RS,2RS).

## Revendications

1 - Le racémique thréo du produit de formule :

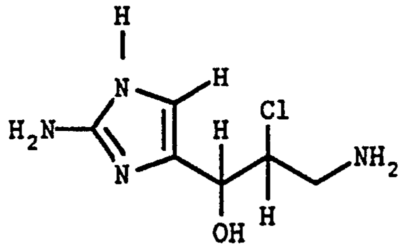

et ses sels.

2 - Procédé de préparation du racémique thréo selon la revendication 1 caractérisé en ce que l'on réduit un dérivé azo racémique thréo de formule générale :

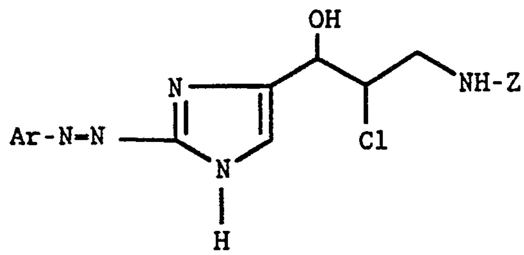

éventuellement sous forme de sel, dans laquelle Ar représente un radical phényle éventuellement substitué par un ou plusieurs atomes ou radicaux, identiques ou différents, choisi parmi les atomes d'halogène et les radicaux alcoyles contenant 1 à 4 atomes de carbone ou alkoxycarbonyles dont la partie alkyle contient 1 à 4 atomes de carbone, ou nitro, et Z représente un groupement protecteur de la fonction amine remplaçable par un atome d'hydrogène par hydrolyse en milieu acide, en passant intermédiairement, selon la nature du groupement protecteur Z par un produit de formule générale :

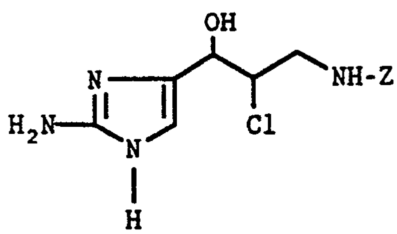

dont on remplace le groupement Z par un atome d'hydrogène, et isole le produit obtenu éventuellement sous forme de sel.

3 - Procédé selon la revendication 2 caractérisé en ce que l'on réduit un dérivé azo de formule générale :

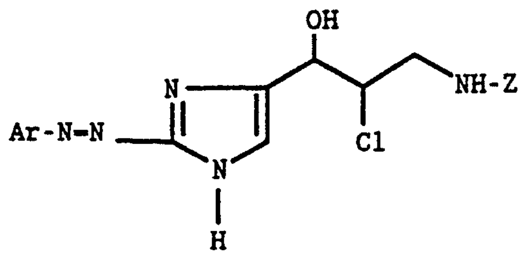

dans laquelle Ar est défini comme dans la revendication 1 et Z représente le radical formyle ou un radical

acyle ou alkoxycarbonyle dont la partie alkyle contient 1 à 4 atomes de carbone, éventuellement substitué par un radical phényle pour obtenir le produit de formule générale :

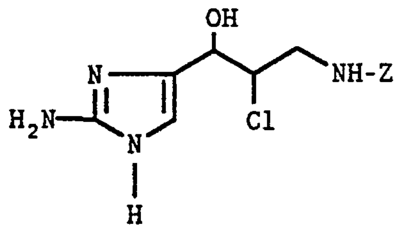

dont on remplace le groupement Z par un atome d'hydrogène par hydrolyse en milieu acide.

4 - Procédé selon l'une des revendications 1 ou 2 caractérisé en ce que la réduction est effectuée au moyen d'hydrogène en présence d'un catalyseur d'hydrogénation.

5 - Procédé selon la revendication 4 caractérisé en ce que le catalyseur est l'oxyde de platine.

6 - Procede selon l'une des revendications 2, 3, 4 ou 5 caractérisé en ce que l'on opère dans un solvant organique.

7 - Procédé selon la revendication 6 caractérisé en ce que le solvant organique est choisi parmi les alcools.

8 - Procédé selon la revendication 7 caractérisé en ce que le solvant est choisi parmi le méthanol et l'éthanol.

9 - Procédé selon l'une des revendications 2, 3, 4, 5, 6, 7 ou 8 caractérisé en ce que la réduction est effectuée à une température comprise entre 0 et 30 °C.

10- Procédé selon l'une des revendications 2 ou 3 caractérisé en ce que le remplacement du groupement Z par un atome d'hydrogène est effectué dans l'eau ou dans un alcool en présence d'un acide minéral.

11 - Procédé selon la revendication 10 caractérisé en ce que l'alcool est choisi parmi le méthanol, l'éthanol, le propanol ou l'isopropanol.

12 - Procédé selon la revendication 10 caractérisé en ce que l'acide est choisi parmi les acides chlorhydrique et sulfurique.

13 - Procédé selon la revendication 10 caractérisé en ce que l'on opère à une température comprise entre 0 et 50 °C.

14 - Procédé de préparation du racémique thréo selon la revendication 1 caractérisé en ce que l'on réduit un dérivé azo racémique thréo de formule générale :

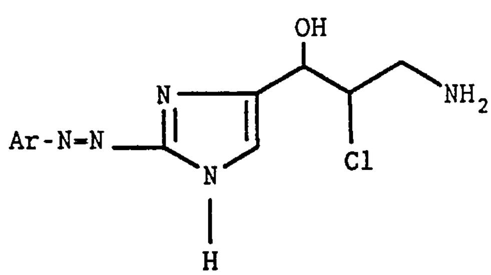

éventuellement sous forme de sel, dans laquelle Ar représente un radical phényle éventuellement substitué par un ou plusieurs atomes ou radicaux, identiques ou différents, choisis parmi les atomes d'halogène et les radicaux alcoyles contenant 1 à 4 atomes de carbone ou alcoyloxycarbonyles dont la partie alcoyle contient 1 à 4 atomes de carbone ou nitro, au moyen d'acide formique en présence de zinc, et isole le produit obtenu éventuellement sous forme de sel.

15 - Procédé selon la revendication 14 caractérisé en ce que l'on opère dans un solvant organique choisi parmi les alcools, les acides organiques ou les solvants chlorés ou un mélange de ces solvants.

16 - Procédé selon l'une des revendications 14 ou 15 caractérisé en ce que l'on opère à une température comprise entre 0 et 25 °C.

17 - Composition pharmaceutique caractérisée en ce qu'elle contient une quantité suffisante du produit selon la revendication 1 en assocation avec un ou plusieurs produits pharmaceutiquement acceptables

inertes ou physiologiquement actifs.

18 - Le produit selon la revendication 1 à titre d'intermédiaire pour la préparation de la girolline.

Revendications pour les Etats contractants suivants: ES, GR

1 - Procédé de préparation du racémique thréo du produit de formule :

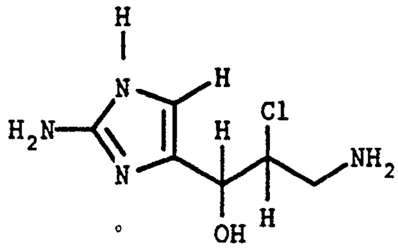

éventuellement sous forme de sel caractérisé en ce que l'on réduit un dérivé azo racémique thréo de formule générale :

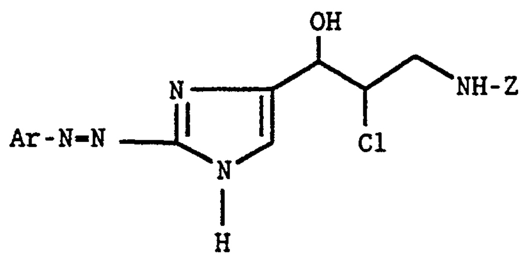

éventuellement sous forme de sel, dans laquelle Ar représente un radical phényle éventuellement substitué par un ou plusieurs atomes ou radicaux, identiques ou différents, choisi parmi les atomes d'halogène et les radicaux alcoyles contenant 1 à 4 atomes de carbone ou alkoxycarbonyles dont la partie alkyle contient 1 à 4 atomes de carbone, ou nitro, et Z représente un groupement protecteur de la fonction amine remplaçable par un atome d'hydrogène par hydrolyse en milieu acide, en passant intermédiairement, selon la nature du groupement protecteur Z par un produit de formule générale :

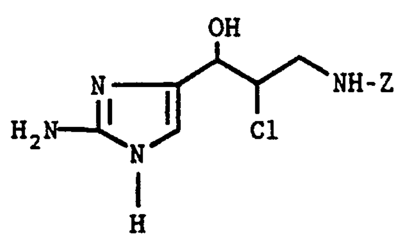

dont on remplace le groupement Z par un atome d'hydrogène, et isole le produit obtenu éventuellement sous forme de sel.

2 - Procédé selon la revendication 1 caractérisé en ce que l'on réduit un dérivé azo de formule générale :

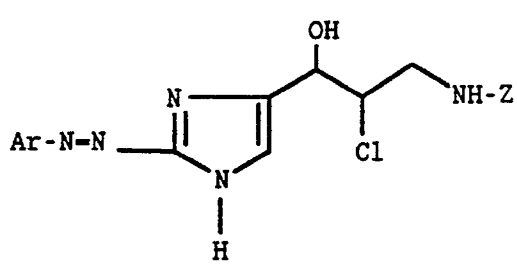

dans laquelle Ar est défini comme dans la revendication 1 et Z représente le radical formyle ou un radical acyle ou alkoxycarbonyle dont la partie alkyle contient 1 à 4 atomes de carbone, éventuellement substitué par un radical phényle pour obtenir le produit de formule générale :

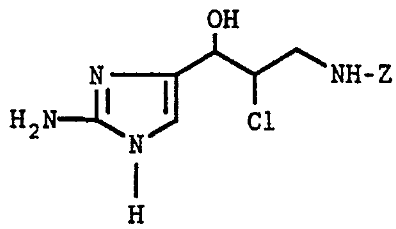

dont on remplace le groupement Z par un atome d'hydrogène par hydrolyse en milieu acide.

3 - Procédé selon l'une des revendications 2 ou 3 caractérisé en ce que la réduction est effectuée au moyen d'hydrogène en présence d'un catalyseur d'hydrogénation.

4 - Procédé selon la revendication 3 caractérisé en ce que le catalyseur est l'oxyde de platine.

5 - Procédé selon l'une des revendications 1, 2, 3 ou 4 caractérisé en ce que l'on opère dans un solvant organique.

6 - Procédé selon la revendication 5 caractérisé en ce que le solvant organique est choisi parmi les alcools.

7 - Procédé selon la revendication 6 caractérisé en ce que le solvant est choisi parmi le méthanol et l'éthanol.

8 - Procédé selon l'une des revendications 1, 2, 3, 4, 5, 6 ou 7 caractérisé en ce que la réduction est effectuée à une température comprise entre 0 et 30° C.

9 - Procédé selon l'une des revendications 1 ou 2 caractérisé en ce que le remplacement du groupement Z par un atome d'hydrogène est effectué dans l'eau ou dans un alcool en présence d'un acide minéral.

10 - Procédé selon la revendication 9 caractérisé en ce que l'alcool est choisi parmi le méthanol, l'éthanol, le propanol ou l'isopropanol.

11 - Procédé selon la revendication 9 caractérisé en ce que l'acide est choisi parmi les acides chlorhydrique et sulfurique.

12 - Procédé selon la revendication 9 caractérisé en ce que l'on opère à une température comprise entre 0 et 50° C.

13 - Procédé de préparation du racémique thréo du produit de formule :

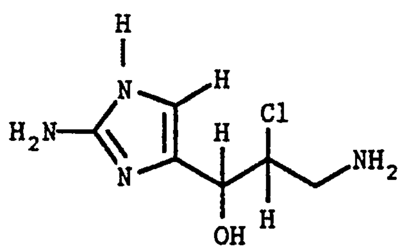

éventuellement sous forme de sel, caractérisé en ce que l'on réduit un dérivé azo racémique thréo de formule générale :

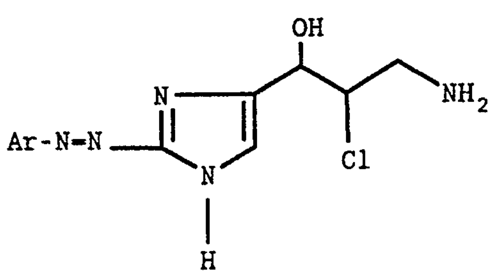

éventuellement sous forme de sel, dans laquelle Ar représente un radical phényle éventuellement substitué par un ou plusieurs atomes ou radicaux, identiques ou différents, choisis parmi les atomes d'halogène et les radicaux alcoyles contenant 1 à 4 atomes de carbone ou alcoyloxycarbonyles dont la partie alcoyle contient 1 à 4 atomes de carbone ou nitro, au moyen d'acide formique en présence de zinc, et isole le produit obtenu éventuellement sous forme de sel.

14 - Procédé selon la revendication 13 caractérisé en ce que l'on opère dans un solvant organique choisi parmi les alcools, les acides organiques ou les solvants chlorés ou un mélange de ces solvants.

15 - Procédé selon l'une des revendications 13 ou 14 caractérisé en ce que l'on opère à une température comprise entre 0 et 25 °C.

Office européen des brevets

# RAPPORT DE RECHERCHE EUROPEENNE

Numero de la demande

EP 90 40 1236

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,A | FR-A-2 585 020 (RHONE-POULENC SANTE)<br>----- | | C 07 D 233/88<br>A 61 K 35/56<br>A 61 K 31/415 |
| | | | **DOMAINES TECHNIQUES RECHERCHES (Int. Cl.5)** |
| | | | C 07 D 233/00<br>A 61 K 35/00<br>A 61 K 31/00 |

Le présent rapport a été établi pour toutes les revendications

| Lieu de la recherche | Date d'achèvement de la recherche | Examinateur |
|---|---|---|
| LA HAYE | 25-07-1990 | DE BUYSER I.A.F. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant